# EUROPEAN PATENT SPECIFICATION

(11) **EP 2 611 452 B1**
(45) Date of publication and mention of the grant of the patent: **24.10.2018**
(21) Application number: 11758028.2
(22) Date of filing: 02.09.2011
(51) Int. Cl.: C12N 5/0775, A61K 35/28, C12M 1/12

(54) **CELL CULTURE SYSTEM FOR BIOREACTOR SCALE-UP OF CELLS**
ZELLKULTURSYSTEM FÜR BIOREAKTORAUFSKALIERUNG VON ZELLEN
SYSTÈME DE CULTURE CELLULAIRE DESTINÉ À AUGMENTER LA PRODUCTION DE CELLULES EN BIORÉACTEUR

(30) Priority: 02.09.2010 US 379712 P
(43) Date of publication of application: 10.07.2013
(73) Proprietor: Life Technologies Corporation, Carlsbad, CA 92008 (US)
(72) Inventor: CAMPBELL, Andrew, Grand Island, New York 14072 (US); CHASE, Lucas, DeForest, Wisconsin 53532 (US); VEMURI, Mohanachari, Frederick, Maryland 21702 (US)
(74) Representative: Weber, Birgit
(86) International application number: PCT/US2011/050426
(87) International publication number: WO 2012/031263

(56) References cited:
- LINDROOS BETTINA ET AL: "Serum-free, xeno-free culture media maintain the proliferation rate and multipotentiality of adipose stem cells in vitro", CYTOTHERAPY, vol. 11, no. 7, 2009, pages 958-972, XP9157099, ISSN: 1465-3249
- CHASE LUCAS G ET AL: "A novel serum-free medium for the expansion of human mesenchymal stem cells", STEM CELL RESEARCH & THERAPY, BIOMED CENTRAL LTD, vol. 1, no. 1, 2 April 2010 (2010-04-02), page 8, XP021085674, ISSN: 1757-6512, DOI: 10.1186/SCRT8
- SCHOP D ET AL: "Expansion of human mesenchymal stromal cells on microcarriers: growth and metabolism", JOURNAL OF TISSUE ENGINEERING AND REGENERATIVE MEDICINE, JOHN WILEY & SONS, US, vol. 4, no. 2, 19 October 2009 (2009-10-19), pages 131-140, XP009135743, ISSN: 1932-6254, DOI: 10.1002/TERM.224 [retrieved on 2009-10-19]

## Description

Pluripotent stem cells, such as mesenchymal stem cells (MSCs), are ideal candidates for the treatment of a variety of human diseases, including myocardial infarction, ischemic stroke, various autoimmune diseases, etc. Exemplary stem cells, including but not limited to, ES or ESCs (embryonic stem cells), ADSCs (adipocyte stem cells), neural stem cells, iPSCs (induced pluripotent stem cells), human embryonic stem cells (hESC), non-human primate ES cells, etc., are useful for downstream therapeutic applications such as cell therapy. Effective cell therapy could potentially require vast amounts of these cells. As cell therapies progress through clinical development, scale and cost of manufacturing become looming issues. For MSC cell types, such as adipose derived stem cells (ADSCs), etc., most clinical trials have been done on a small scale, where the required cell number was achieved through traditional cell culture techniques where cells grow adherently in 2D on treated tissue culture surfaces. However, these standard 2D culture methods are too labor intensive and inefficient to meet the potential large scale needs of several cell therapy applications. Looking ahead toward scale-up and commercialization, it is likely that approximately 10¹² - 10¹⁴ or more cells, derived from a single allogenic donor, which may be required for effective therapeutic use, especially during treatments requiring multiple doses. This magnitude of MSC cells is far greater than what can be delivered using current manufacturing protocols. More importantly, for human cell therapy, MSC cell culture systems need to completely exclude animal serum during culture for a regulatory compliant end-product.

So far, MSC have been grown on a variety of systems, including 2D cultures which are small scale and microcarriers, using serum, or reduced serum (Dos Santos F. et al., J Cell Physiol 223: 27-35, (2010)). At the time of this filing, it was believed that MSCs would not to attach to microcarriers unless the media contained at least some serum, and/or, at least on microcarriers pre-coated with serum. However, the presence of even minute quantities of serum during MSC culturing is not GMP compliant. Another vital question arising during these MSC cultures in serum is whether such MSCs will stay pluripotent (or maintain their sternness), since serum contains variable differentiating factors in different serum lots which may cause culture differentiation.

Therefore, despite the existence of systems for growing MSCs in culture, there remain many hurdles in obtaining large - scale systems for MSC growth which are GMP-compliant, maintain their sternness, and, are effective for animal therapeutic use.

### SUMMARY OF THE INVENTION

The invention is directed to a method for cultivating a mesenchymal stem cell (MSC) or an induced pluripotent stem cell (IPSC) to high density in suspension comprising: culturing the MSC or IPSC on a microcarrier bead coated with a xeno free-cell attachment substrate with a cell culture medium suitable for the growth and expansion of said MSC or IPSC, under controlled conditions of pH 7.2, 5% actively pumped CO₂, at 37°C temperature, 40 rpm agitation, 20% atmospheric pressure dissolved O₂, wherein said cell culture medium is serum free. In certain embodiments the cell attachment substrate is CELLStart™, or the MSC is an adipocyte derived stem cell (ADSC), or the MSC is derived from an autologous or an allogeneic donor, or in some aspects, the cell is an IPSC.

In another aspect, the disclosure is directed to a composition comprising: i) a SF mesenchymal cell culture medium; ii) a microcarrier bead; iii) a XF cell attachment substrate; and, iv) a MSC. In certain embodiments, the cell attachment substrate is CELLStart™, or the MSC is an adipocyte derived stem cell (ADSC), or the MSC is derived from an autologous or an allogeneic donor.

Generally, the invention is directed to a method for cultivating a MSC comprising growing the MSC on a microcarrier bead coated with a XF cell attachment substrate in a SF mesenchymal cell culture medium under controlled conditions suitable for the growth and expansion of said MSC.

In a further aspect, the disclosure is directed to a method for producing a protein comprising the method described above, wherein the protein is recovered from the MSC following cultivation. In certain embodiments, the cell attachment substrate is CELLStart™, or the MSC is an adipocyte derived stem cell (ADSC), or the MSC is derived from an autologous or an allogeneic donor, or the MSC is obtained either from bone-marrow, blood, skin, cord blood or perichondrium; and to a method described above wherein the protein is a recombinant protein and the MSC is engineered to produce the recombinant protein.

In yet another aspect, the invention is directed to a method for cultivating a MSC to high density in suspension culture comprising: attaching the MSCs to a microcarrier coated with a xeno-free cell attachment substrate; and growing the MSC in a serum-free culture medium that supports the growth of said cells, under conditions suitable for the growth and expansion of said MSC cell.

The disclosure is also directed to a kit for the large-scale cultivation of a stem cell in suspension *in vitro,* said kit comprising one or more containers, wherein a first container contains a serum-free medium in which the stem cell can be cultivated; a second container comprising a xeno-free cell attachment substrate; and the stem cell.

The disclosure is further directed to uses of an MSC obtained by the above mentioned methods, or a use of a recombinant protein obtained from a MSC; or use of a beneficial factor obtained from a stem cell, all for treating a given disease condition.

### Description of Figures

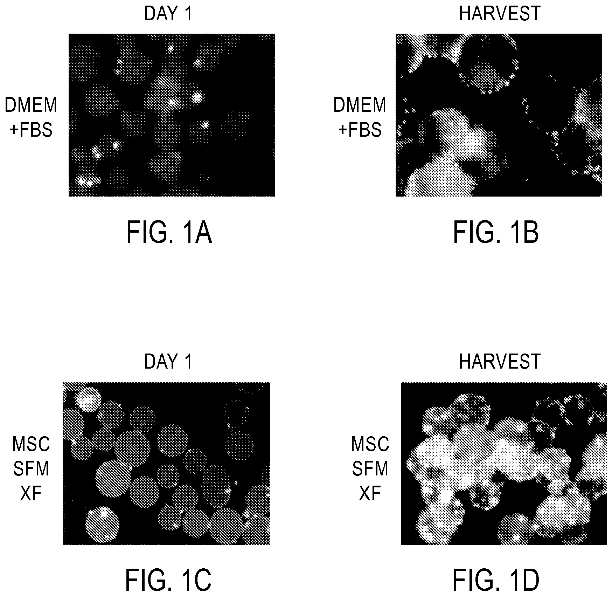

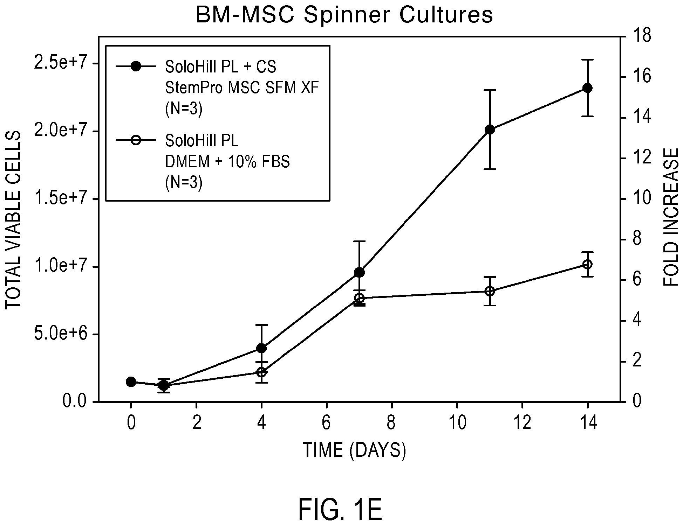

Figure 1: Cell growth kinetics on microcarriers using serum-free and xeno-free (SF and XF) system. Serum-free (SF) microcarrier based MSC expansion was done using StemPro® MSC SFM Xeno-free (XF) medium (see examples). Figures 1A and 1B show MSCs cultured in medium containing 10% FBS and Figures 1C and 1D show MSCs cultured in StemPro® MSC SFM Xeno-free (XF) medium. Approx. 15 fold expansion of MSC cells was observed in StemPro® MSC SFM Xeno-free (XF) medium in 100 ml spinner flasks (also see graph in Figure 1E).

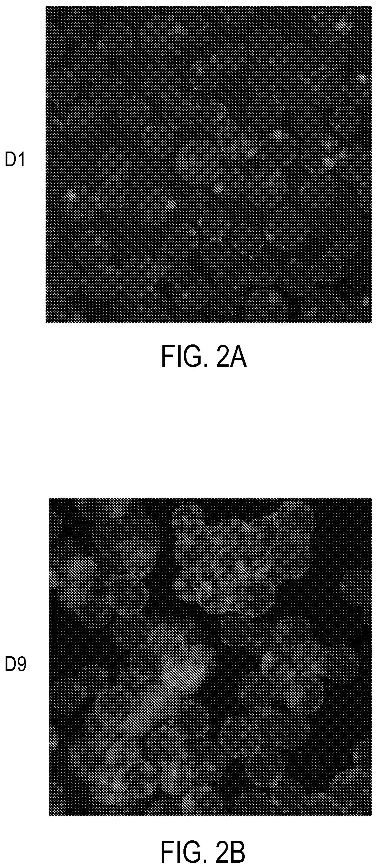

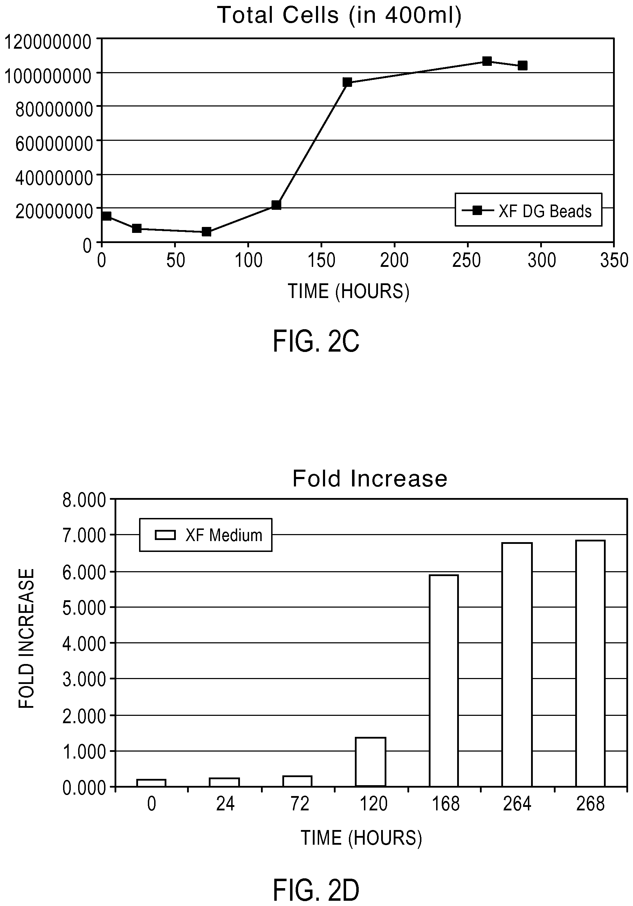

Figure 2: Results from a DasGIP Bioreactor run with StemPro® MSC SFM, Xeno-free (XF) medium. MSC cells were seeded with 37,500 cells/ml in 400 ml volume of SFM/XF medium, and grown on SoloHill 125 micron plastic beads coated with CELLStart™ from Invitrogen. 50% exchange of medium was done every other day. Figure 2A shows cells on beads on day 1, and Figure 2B shows cells on beads on day 9. Cell growth is shown in Figure 2C for the DasGIP (DG beads) Bioreactor run. As seen in Figure 2D, the fold increase in cell number was significant day 5 (120 h), was dramatic by day 7 (168 h) - 6000 fold increase and continued to increase day 12 (288 h).

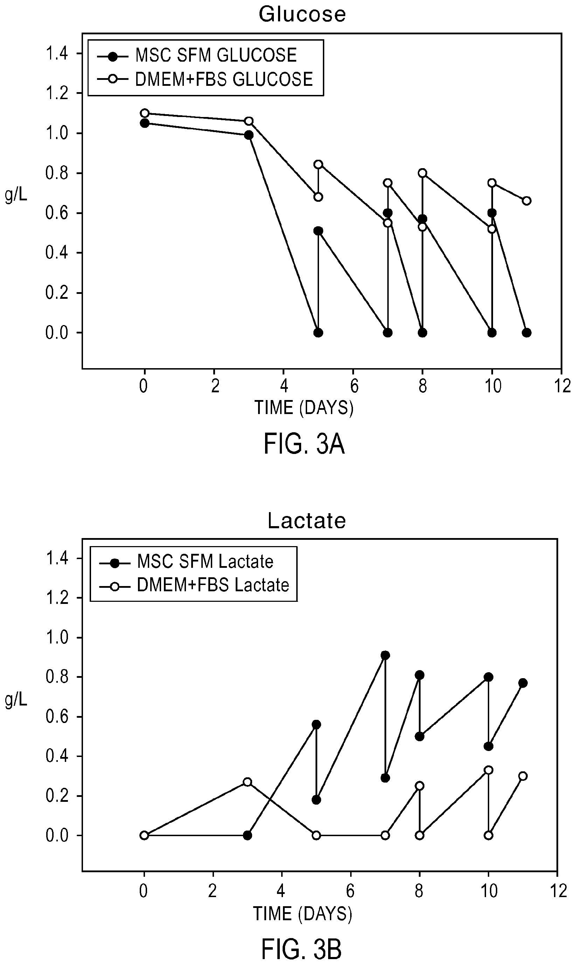

Figure 3: Spent media analysis of bone marrow MSCs grown on microcarriers in StemPro® MSC SFM, Xeno-free (XF) medium. Panel 3A shows that glucose was consumed rapidly between 5-11 days. 50% medium was exchanged every 2-3 days. Panel 3B shows that lactate correspondingly accumulated rapidly between 3-11 days.

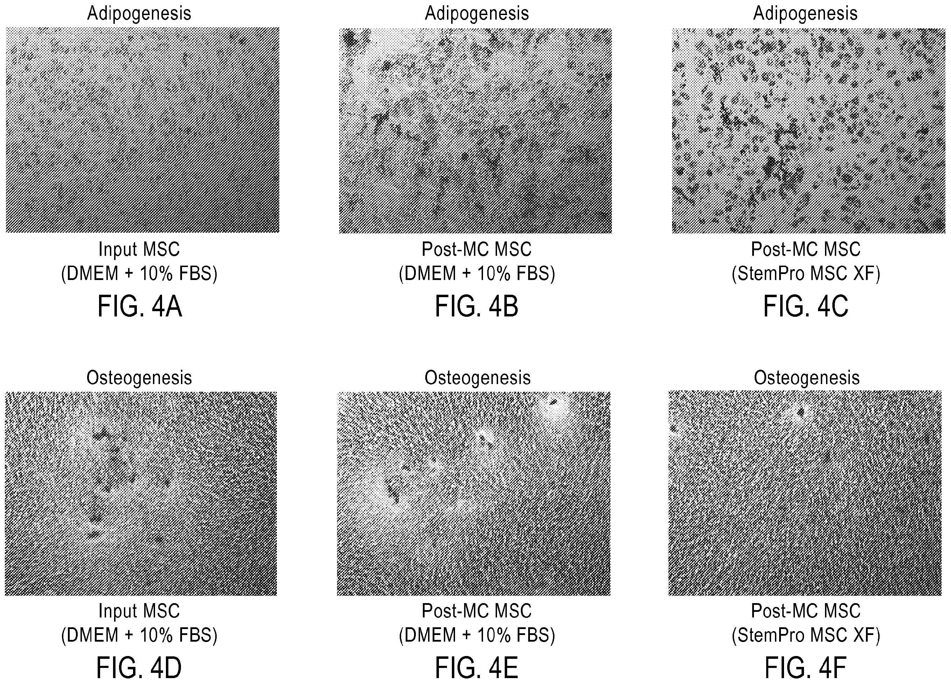

Figure 4: Functional Analysis of MSC cells post bioreactor expansion using cell staining methods. Panels 4A-C: Staining with 0.5% Oil Red O solution in 60% isopropanol for adipogenic staining. 4A: MSCs grown in DMEM medium with 10% MSC-qualified FBS before microcarrier culture; 4B: DMEM medium with 10% MSC-qualified FBS after microcarrier culture; 4C: StemPro® MSC SFM Xeno-free medium microcarrier grown culture showing comparable adipogenic differentiation. Panels 4D-F: Staining cells with Alizarin Red stain for osteogenesis. 4D: MSCs grown in DMEM medium with 10% MSC-qualified FBS before microcarrier culture; 4E: DMEM medium with 10% MSC-qualified FBS after microcarrier culture; 4F: StemPro® MSC SFM Xeno-free medium microcarrier grown culture showing comparable osteogenic differentiation.

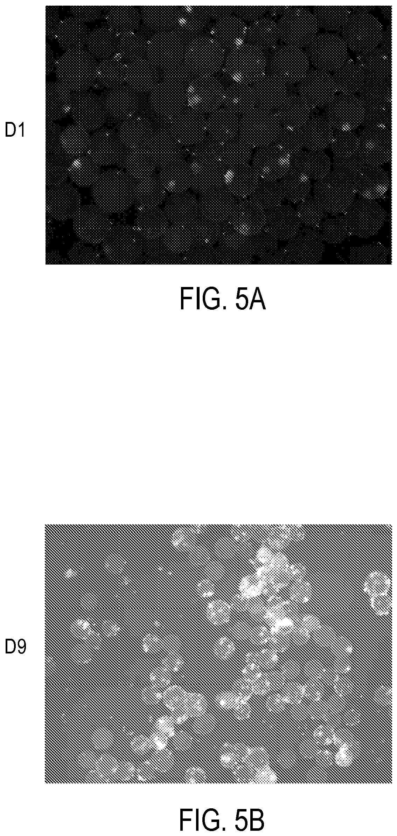

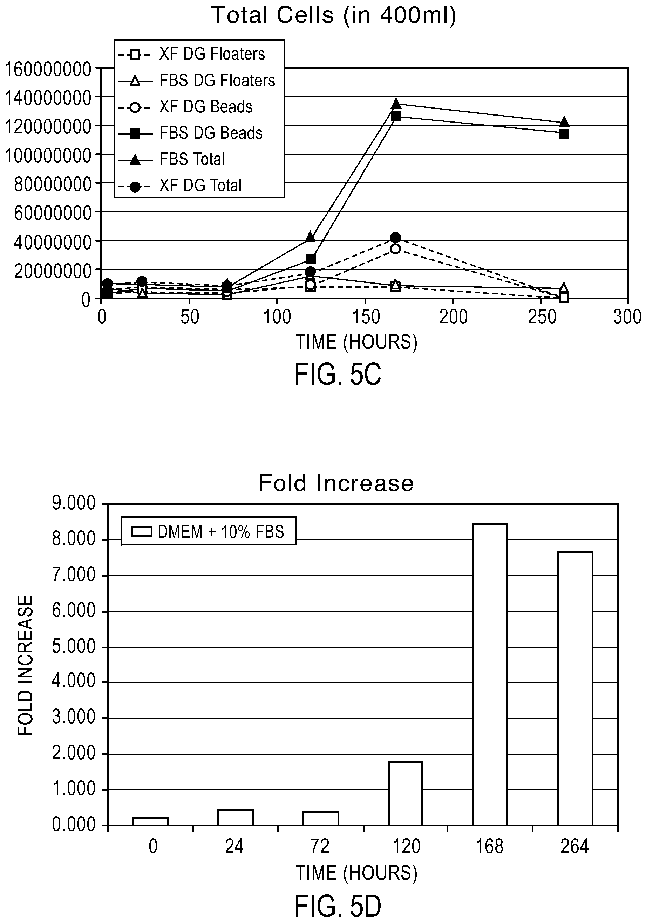

Figure 5: Comparative data of cell growth kinetics on microcarriers using serum containing media (DMEM with 10% FBS) as in Figure 1.

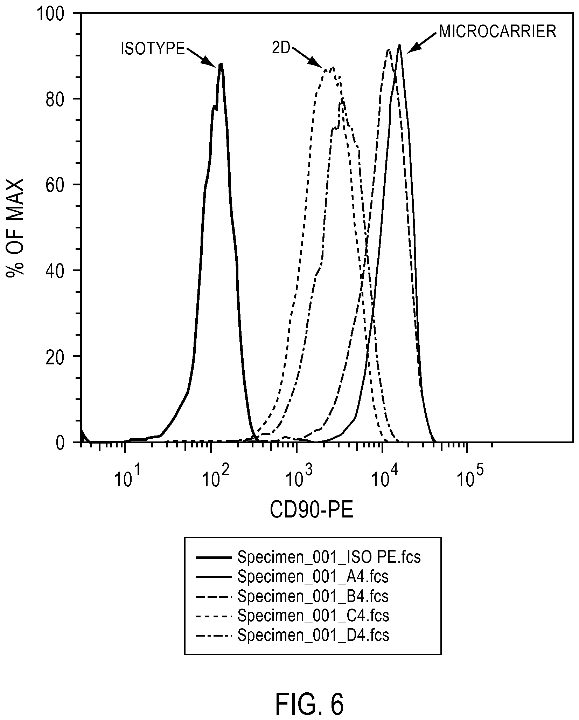

Figure 6: Partial Characterization of MSCs from 2D tissue culture plates and microcarrier cultures using flow cytometry. Shown here is the expression of a positive MSC cell surface marker, CD90 (clone 5E10), in the 2D and microcarrier culture.

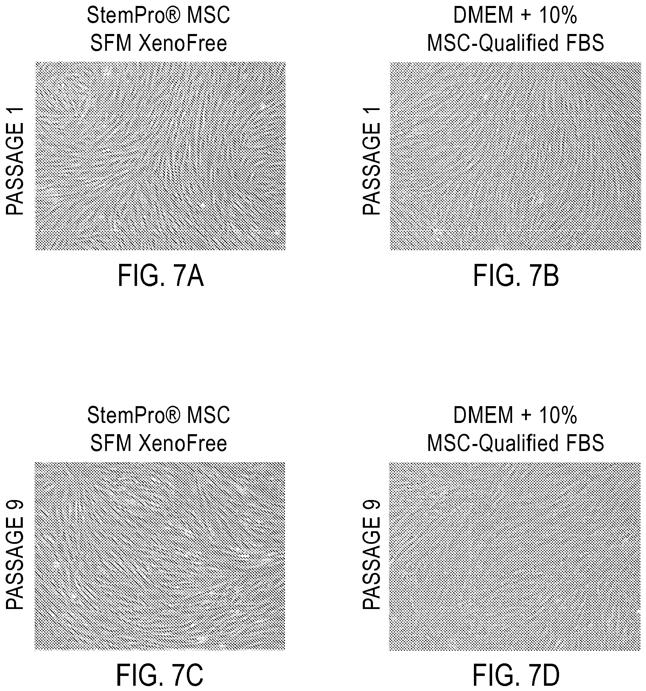

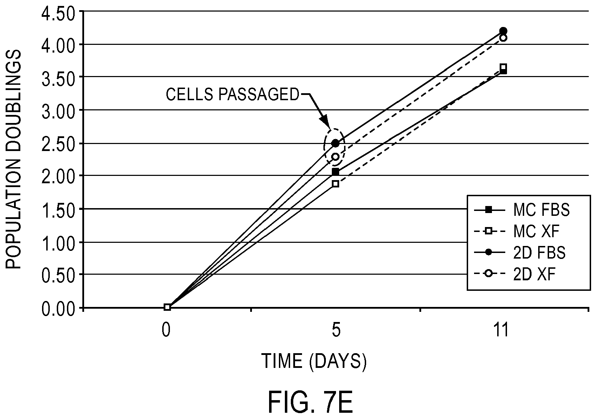

Figure 7: Comparison of human bone marrow MSCs cell cultures in 2D culture. Panels A and C: Cells grown in StemPro® MSC SFM Xeno-free (XF) medium (passage 1 and passage 9, respectively). Panels B and D: Cells grown in DMEM medium with 10% MSC-qualified FBS (passage 1 and passage 9, respectively). Panel 7E shows comparable population doublings of cells grown in 2D and microcarrier cultures, with FBS or under XF conditions.

### Detailed Description

The present disclosure is directed to compositions and methods for cultivating stem cells, such as MSCs, in large-scale culture under serum-free and xeno-free conditions. Most of the embodiments and examples in the application refer to the use of MSC cell cultures for large-scale, serum-free and xeno-free expansion in a cell culture. However, as one of skill in the art may recognize, the teaching of this invention may be applied to a variety of cells, including other known stem cells and stem cell lines. One of skill in the art would know how to practice the invention, or to grow a particular stem cell (including MSCS, ESCs, etc.), by using the corresponding serum-free and xeno-free culture medium. Accordingly, besides MSCs, the compositions and methods of the instant invention may apply to other stem cells, including but not limited to, ES or ESCs (embryonic stem cells), ADSCs (adipocyte stem cells), neural stem cells, iPSCs (induced pluripotent stem cells), human embryonic stem cells (hESC), non-human primate ES cells, etc.

Mesenchymal stem cells (MSCs) are exemplary stem cells that are multipotent mesoderm-derived progenitor cells. They have the capacity to differentiate into a variety of cell types, including but not limited to, adipose or fat cells (adipocytes), bone (osteocytes), cartilage (chondrocytes), tendons (tenocyte), or muscle tissue (myoblasts), etc., presenting a wide potential for the development of cell-based therapies. Adult mesenchymal stem cells can be isolated from the stroma of the bone marrow, or from alternative sources which include, but are not limited to, blood, skin, cord blood, and perichondrium. Hence, MSCs may alternately be referred to as bone marrow stromal stem cells or skeletal stem cells.

Mesenchymal stem cells may transdifferentiate into other lineage cells, including but not limited to, neural, hepatic, endothelial cells, etc.

MSCs may be useful in the repair or regeneration of several damaged tissues or conditions in bone, cartilage, meniscus or myocardial tissues, etc. Several investigators have used MSCs with encouraging results for transplantation in a variety of animal disease models including but not limited to, osteogenesis imperfecta, spinal cord injury, bone marrow transplants, myocardial infarction, ischemic stroke, etc., and recently, with various immune diseases. MSCs have been associated with the treatment of immune diseases, including but not limited to, disorders involving inflammation, epithelial damage, psoriasis, or allergic responses, autoimmune diseases, arthritis, inflamed wounds, alopecia araeta (baldness), periodontal diseases including gingivitis and periodontitis, and other diseases.

Mesenchymal stem cell markers: Undifferentiated mesenchymal stem cell may be identified by specific markers, which can be identified with unique monoclonal antibodies. Undifferentiated mesenchymal stem cell markers include, but are not limited to, intracellular markers like nucleostemin, and also cell surface markers, including but not limited to, bone morphogenetic proteins (BMPR), Endoglin, Stem Cell Factor Receptor (SCF R), STRO-1, CD90, etc.

The mesenchymal stem cell may be obtained from a spectrum of sources including autologous (individual's own) or allogeneic (stem cell from a matching donor) sources.

Provided herein are methods and compositions related to culturing stem cells like MSCs in a large-scale, serum-free and xeno-free (free of substances from any animal other than the species of animal from which the stem cells are derived) system, on microcarriers in any suitable cell culture container, such as a tissue culture flask, or preferably, in a container where MSCs can be cultivated in a large-scale, such as, a bioreactor. Microcarrier based culture systems in traditional stirred-tank bioreactors have the potential to generate large cell numbers that may be sufficient for the production of many thousands of doses of MSC cells or MSC cell products. A relevant scale-up solution for the production of mass quantities of cells, etc., would reduce the cost and time of producing cell products. This would enable the large-scale manufacturing of cell therapy products for clinical use.

Serum-free culture conditions are sought for the high density culture of clinical-grade MSCs, as serum is a mixture of many components, and although it is relatively well-characterized, sera can vary from lot to lot. Also, sera contains factors that can cause differentiation of stem cells, thereby resulting in loss of their stem characteristics (sternness). Xeno-free culture conditions are further sought for the generation of these high density, clinical-grade MSC cultures. For example, commonly used animal proteins like albumin collagen, cannot be used for cell attachment to tissue culture flasks or microcarriers under xeno-free conditions; only human derived proteins can be used. In addition, novel stem cell AOF (animal origin free) media and feeds are being developed that support prolonged MSC proliferation while maintaining MSC identity to meet requirements in this field (*e.g*., StemPro Efficient Feeds).

One tool that may be of use in the culture of these clinically relevant systems would be a simple, defined cell (*e*.*g*., stem cell) matrix that has useful characteristics that approximate, or preferably, improve upon and/or increase the number of, cells on the microcarriers, thereby increasing the production of many thousands of doses of cell product. In conjunction with certain media, such a matrix could be used, for example, to generate stem cell cultures that are defined, serum-free and/or xeno-free. Such matrices may also be used with other cell types as well. For example, non-stem cells which exhibit enhanced growth characteristics when in contact with a matrix.

According to an aspect of the invention, the methods and compositions of the system use microcarriers. By "microcarrier" is meant a microcarrier bead, where the cell to be cultured is cultured on the surface of the microcarrier bead within a cell culture container, like a flask, tissue culture dish, or a bioreactor. In microcarrier culture, cells may grow as monolayers on the surface of small spheres, or as multilayers within the pores of a macroporous structure in the bead. Microcarriers may be usually suspended in culture medium by gentle stirring. By using microcarriers in simple suspension culture, fluidized or packed bed systems may yield up to 200 million cells per milliliter. Exemplary microcarrier beads include, but are not be limited to, Solohill beads, Glass beads, Cytodex® microcarrier beads, Hillex®, etc.

The microcarrier beads may be uncoated or coated with a variety of coatings, for example, with recombinant protein coatings, animal protein coating (for e.g., collagen, gelatin, charged gelatin, etc.), that would be specific to the type of cell to be cultured. Protein-free microcarriers are available for culture as well, for example, glass, plastic, Hillex®, etc. In a preferred embodiment, the microcarrier bead is coated with CELLStart™ (Invitrogen), which is a proprietary, xeno-free, human-derived substrate used for the attachment and expansion of human embryonic, mesenchymal, and neural stem cells.

In another aspect of the invention, suitable cell containers are used to maintain the microcarriers in suspension for cell growth. Suitable cell culture containers may include, but not be limited to, a bioreactor (for e.g., DasGIP bioreactor) that comprises equipment which gives even suspension of the microcarriers with gentle stirring, and allows for adequate exchange of gases with the culture medium. Erratic stirring motions may not be desirable since they could lead to cell detachment of rounded cells like mitotic cells from the microcarriers. The shape of the culture vessel and stirring mechanism may be designed to prevent sedimentation of microcarriers in any part of the culture vessel. In one embodiment, a cell culture container with slightly rounded bases may be preferred.

In some embodiments, a controlled bioreactor may be used. Some exemplary conditions that may be controlled for stem cell growth are pH (from about 6.5 to 7.5, more preferably from about 7.0 to 7.5, most preferably pH 7.2, etc.); actively pumped CO₂ (from about 3-10%, preferably from about 5-8%, more preferably about 5%, etc.); temperature (from about 30°C to 40°C, more preferably about 37°C); agitation (from about 30-45 rpm, more preferably about 40 rpm); dissolved O₂ (from about 3% to 20% atmospheric O₂, more preferably about 5% to 20% atmospheric O₂, most preferably about 20% atmospheric O₂). In a preferred embodiment, SF and XF MSC cultures were grown in a controlled bioreactor at pH 7.2, 5% actively pumped CO₂, 37°C, agitation at 40 rpm, dissolved O₂ 20% atmospheric. The pH of the medium may be maintained due to auto-pHing buffers in the medium as are well known in the art, such as sodium bicarbonate.

In some embodiments, suitable cell culture reagents may include any reagent useful for the cultivation, growth, multiplication of cells including basal serum-free, xeno-free media, reconstituted media, xeno-free media supplements, vitamins, xeno-free growth factors, etc.

When trying to scale up adherent cell types there are at least three options: 1) modify cells to grow in suspension, 2) scale-out through multiple parallel small scale cultures, or 3) create a larger surface area within the same volume. Modifying cells to grow in suspension is possible, but may change the characteristics and/or function of the cell and needs to be re-engineered for each cell type. Scale-out is also possible, but does not allow for economies of scale and eventually becomes impractical due to space and cost considerations. Creating an environment for the scale- up of adherent cell culture through a microcarrier based system, however, may avoid one or more of these disadvantages and may provide a low cost, regulatory compliant method for the large scale expansion of adherent cell types for therapeutic purposes. This process for cells such as MSCs and ADSCs encompasses not only the microcarrier and method, but also potentially includes changes to media and reagents to allow them to grow high density culture, systems to remove and purify cells from carriers, and ensuring genetic and phenotypic stability of the cells while maintaining proliferative potential.

The stem cells obtained by the serum-free and xeno-free conditions described above may have many uses, for example, in the treatment of disease conditions, or for other downstream therapeutic use, including but not limited to, stem cell therapy, or the production of products, such as beneficial factors, obtained from the stem cells of the invention. "Beneficial factors" derived from the cells may include, but are not limited to, recombinant proteins, growth factors, wound healing promoting factors or secretions, cytokines, matrix components, cell attachment factors, etc.

In accordance with a use for the MSCs, a method of treating a disease or condition in an animal is also provided. The method comprises administering to the animal mesenchymal stem cells in an amount effective to treat disease or condition in the animal. To do so, the mesenchymal stem cells may be administered in an amount effective to treat or alleviate the disease or condition. Accordingly, mesenchymal stem cells may be administered in an amount of from about 1 X 10⁵ cells/kg to about 1 X 10⁷ cells/kg. In another embodiment, the mesenchymal stem cells may be administered in an amount of from about 1 X 10⁶ cells/kg to about 5 X 10⁶cells/kg. The amount of mesenchymal stem cells to be administered may depend upon a variety of factors, including the age, weight, sex of the individual/patient to be treated, and to the extent or severity of the disease or condition.

The mesenchymal stem cells may be administered in conjunction with an acceptable pharmaceutical carrier. For example, the mesenchymal stem cells may be administered as a cell suspension in a pharmaceutically acceptable liquid medium or gel for injection or topical application.

As one of skill in the art would also know, the mesenchymal stem cells may be administered by a variety of procedures, for instance, systemically, such as by intravenous, intraarterial, or by intraperitoneal administration.

It should be understood that while the present teachings have been described in detail with respect to various exemplary embodiments thereof, it should not be considered limited to such, as numerous modifications are possible without departing from the broad scope of the appended claims.

The specific examples below are to be construed as merely illustrative, and not limitative of the remainder of the disclosure in any way whatsoever. Without further elaboration, it is believed that one skilled in the art can, based on the description herein, utilize the present invention to its fullest extent. Further, any mechanism proposed below does not in any way restrict the scope of the claimed invention.

### Examples

### Example 1: Growth and Expansion of MSCs in Stirred-tank system: Spinner flasks (100 ml) and in DasGIP bioreactors (400 ml).

Human bone marrow derived MSCs were utilized to look at the adaptation of cells from traditional 2D culture into a 3D culture. Data is shown here of the growth of human bone marrow-derived MSCs on microcarriers in a stirred-tank system (small scale spinner flasks and in 500ml bioreactors), using regulatory-friendly xeno-free media and reagents.

Initial studies were conducted to evaluate the StemPro® MSC SFM Xeno-Free medium (Invitrogen, cat. A10675-01) for their ability to support MSC expansion on microcarriers. The protocol for XF cell culture were adapted internally from MSC growth protocols using serum-containing systems obtained by courtesy of the Cabral group, Institute for Biotechnology and Bioengineering (IBB), Avenida Rovisco Pais, Lisboa, Portugal. MSC cells were grown on SoloHill 125 micron, plastic beads (SoloHill Engineering Inc., cat. P102-1521) and pre-coated with the xeno-free attachment factor CELLStart™ (Invitrogen, cat. A1014201). Cells were seeded at 25,000 cells/ml. Cell cultures were initiated in 100ml spinner flasks (Bellco® spinner flasks from Bellco Glass, Inc. with 90° paddles (normal paddles) and a magnetic stir bar and growth was compared with traditional cell culture media (DMEM + 10% FBS). 50% complete medium was fed every 2-3 days. Figures 1A, 1B, 1C and 1D depict these results over a 14 day assay and bone marrow derived MSCs grown on microcarriers were at least comparable to cells grown in 2D culture. Cells were harvested from the microcarriers using the animal-origin-free dissociation reagent TrypLE (Invitrogen, cat. 12604039). Cell number and viability were determined using the Trypan Blue exclusion method using a ViCell counter. Cell expansion was approximately 15 fold in the XF formulation compared to the DMEM + FBS control medium in the small scale, spinner flask system (Figure IE).

The above small scale experiments were confirmed in a 500ml DasGIP stirred-tank bioreactor (400ml working volume), using the same SoloHill plastic microcarrier beads pre-coated with the xeno-free attachment factor CELLStart™, and the StemPro® MSC SFM Xeno-Free medium. Process parameters were controlled as follows: pH - 7.2, carbon dioxide actively pumped at 5%, temperature - 37°C, agitation at 40 rpm, controlled dissolved oxygen (atmospheric or 20%). The cultures were fed 50% fresh medium every 2-3 days. As shown in Figures 2A-D, the xeno-free medium enabled cell expansion from an initial seeding density of 37,500 cells/ml to approximately 262,000 cells/ml. This represents a total of ∼105 million cells in total, for the bioreactor run. This data shows the feasibility of using serum-free microcarrier expansion of MSCs using the XF media and microcarrier system in a bioreactor. Further large scale volumes are also being checked.

### Example 2: Functional Analysis of MSC cells post expansion (Cell Staining Methods from Chase et al. Stem Cell Research & Therapy 2010, 1:8).

MSC cells were analyzed further for cell growth kinetics, cell phenotype, spent media analysis for glucose consumption and lactate production (Figure 3A and 3B respectively), and for functionality post culture in the bioreactor. Glucose was consumed rapidly between days 5-11. Medium exchange or glucose feed is necessary. These characteristics of the MSCs that were obtained were measured by their ability to differentiate along a certain differentiation pathway, for example, osteogenic, chondrogenic or adipogenic lineages.

To evaluate this, cells were harvested from the microcarriers using the animal-origin-free dissociation reagent TrypLE (Invitrogen, cat. 12604039). MSCs were set up in standard culture conditions for osteogenic, chondrogenic and adipogenic differentiation. Cells were plated in 12-well plates as per the manufacturer's protocol and induced to differentiate down adipogenesis and osteogenisis pathways using commercially available kits (Invitrogen, Human Mesenchymal Stem Cell (hMSC) Differentiation Kits: Osteogenesis, cat. A10072-01; Adipogenesis, cat. A10070-01). During differentiation, complete medium changes were made every 3 to 4 days. After 14 days, cultures were monitored for differentiation by using lineage-specific biologic stains (Figure 4, A-F). After staining, cultures were washed with 0.1N HCl and visualized. To monitor adipogenic differentiation, cultures were stained with Oil Red O (Sigma) (0.5% Oil Red O solution in 60% isopropanol), washed with distilled water, and visualized (Figure 4, A-C). For chondrogenic differentiation, micromass pellet cultures were fixed with 4% formaldehyde and stained with 1% Alcian Blue (Sigma) (not shown). For osteogenesis, after 21 days of differentiating conditions, cells were stained with Alizarin Red stain (Figure 4, D-F). MSCs harvested from the bioreactor differentiated into osteogenic, chondrogenic and adipogenic lineages and were stable on microcarriers for 14 days (2 weeks).

### Example 3: Partial Characterization of MSCs from 2D and microcarrier cultures using flow cytometry (protocols from Chase et al. Stem Cell Research & Therapy 2010, 1:8).

MSCs expanded in serum-containing medium (SCM) or serum-free medium (SFM) on microcarrier cultures or tissue culture plates (2D) were harvested by using TrypLE™ Express (Invitrogen), and washed with DPBS (without Ca^{2+/}Mg²⁺) supplemented with 5% FBS, and stained with the following antibodies: CD11b (unconjugated, clone VIM12), CD34-APC (clone 581), CD45-AF405 (clone HI30), CD44-PE (clone MEM-85), CD105-APC (clone SN6), (all from Invitrogen); CD90 (clone 5E10) - the positive MSC cell surface marker (shown in Figure 6), CD73-PE (clone AD2), (both from BD Biosciences); and where unconjugated primary antibodies were used, AlexaFluor® 488 anti-mouse (Invitrogen) was used. To set background fluorescence levels, unstained and/or matched isotype controls were used.

The data suggests that large scale culture of MSC-like cells using microcarriers is feasible, cost effective and can be done within a closed system, with a higher degree of process control and minimal footprint. Microcarrier expansion under xeno-free conditions is a viable alternative to traditional 2D cell culture methods. Further developments of animal-origin-free microcarrier systems (including the absence of human proteins) are in progress. In addition, specific MSC growth factor and nutrient feed concentrates can be incorporated into the bioreactor systems to provide an efficient fed-batch production model.

## Claims

1. A method for cultivating a mesenchymal stem cell (MSC) or an induced pluripotent stem cell (IPSC) to high density in suspension comprising:
culturing the MSC or IPSC on a microcarrier bead coated with a xeno free-cell attachment substrate with a cell culture medium suitable for the growth and expansion of said MSC or IPSC, under controlled conditions of pH 7.2, 5% actively pumped CO₂, at 37°C temperature, 40 rpm agitation, 20% atmospheric pressure dissolved O₂,
wherein said cell culture medium is serum free.

2. The method of claim 1, wherein the MSC is an adipocyte derived stem cell (ADSC).

3. The method of claim 1, wherein the MSC is derived from an autologous or an allogeneic donor.

4. The method of claim 1, wherein the MSC was obtained either from bone-marrow, blood, skin, cord blood or perichondrium.

5. The method of claim 1, wherein the MSC maintains osteogenic, chondrogenic and adipogenic lineage differentiation potential to day 14.

## Patentansprüche

1. Verfahren zum Kultivieren einer mesenchymalen Stammzelle (MSC) oder einer induzierten pluripotenten Stammzelle (IPSC) zu hoher Dichte in Suspension, umfassend:
Kultivieren der MSC oder IPSC auf einem Mikroträger-Kügelchen, der mit einem xenofreien Anheftungssubstrat beschichtet ist,
mit einem Zellkulturmedium, das für das Wachstum und die Expansion der MSC oder IPSC geeignet ist, unter kontrollierten Bedingungen von pH 7,2, 5% aktiv gepumptem CO₂ bei einer Temperatur von 37 °C, 40 U/min Rühren, 20% bei Atmosphärendruck gelöstem O₂,
wobei das Zellkulturmedium frei von Serum ist.

2. Verfahren nach Anspruch 1, wobei die MSC eine aus Adipocyten abgeleitete Stammzelle (ADSC) ist.

3. Verfahren nach Anspruch 1, wobei die MSC eine von einem autologen oder allogenen Donor abgeleitete MSC ist.

4. Verfahren nach Anspruch 1, wobei die MSC entweder von Knochenmark, Blut, Haut, Nabelschnurblut oder Perichondrium erhalten wurde.

5. Verfahren nach Anspruch 1, wobei die MSC bis zum Tag 14 osteogenes, chondrogenes und adipogenes Potential zur Abstammungsdifferenzierung bewahrt.

## Revendications

1. Procédé de culture d'une cellule souche mésenchymateuse (CSM) ou d'une cellule souche pluripotente induite (CSPI) à haute densité en suspension comprenant :
la mise en culture de la CSM ou de la CSPI sur une bille micro-porteuse revêtue d'un substrat d'attachement cellulaire exempt de xéno-contaminants avec un milieu de culture cellulaire adapté à la croissance et à l'expansion de ladite CSM ou CSPI, dans des conditions contrôlées à un pH de 7,2, à 5 % de CO₂ activement pompé, à une température de 37° C, à une agitation de 40 tr/min, à de l'O₂ dissous à une pression atmosphérique de 20 %,
dans lequel ledit milieu de culture cellulaire est exempt de sérum.

2. Procédé selon la revendication 1, dans lequel la CSM est une cellule souche dérivée d'un adipocyte (CSDA).

3. Procédé selon la revendication 1, dans lequel la CSM est dérivée d'un donneur autologue ou allogénique.

4. Procédé selon la revendication 1, dans lequel la CSM a été obtenue à partir de moelle osseuse, de sang, de peau, de sang ombilical ou de périchondre.

5. Procédé selon la revendication 1, dans lequel la CSM maintient un potentiel de différenciation de lignée ostéogène, chondrogène et adipogène au 14^{e} jour.
